# EUROPEAN PATENT SPECIFICATION

(11) **EP 0 491 103 B1**
(45) Date of publication and mention of the grant of the patent: **20.03.1996**
(21) Application number: 90500123.6
(22) Date of filing: 19.12.1990
(51) Int. Cl.: A61K 31/28, A61K 31/80

(54) **Compositions for therapeutic use comprising organogermanium derivatives**
Organogermaniumderivate enthaltende Zubereitungen zur therapeutischen Verwendung
Compositions pour l'application thérapeutique contenant des dérivés d'organogermanium

(43) Date of publication of application: 24.06.1992
(73) Proprietor: Larranaga Mendizabal, Eusebio, E-20730-Azpeitia (Gipuzkoa) (ES); Duffaut, Norbert Charles, F-33720 Podesac, Bordeaux (FR); Dunogues, Jacques Paul, F-33400 Talence, Bordeaux (FR); Picard, Jean Paul, F-33400 Talence, Bordeaux (FR); Ganboa Landa, José Ignacio, E-20730 Azpeitia (Gipuzkoa) (ES); Aizpurua Iparraguirre, Jesus Maria, Usurbil, (Gipuzkoa) (ES)
(72) Inventor: Larranaga Mendizabal, Eusebio, E-20730-Azpeitia (Gipuzkoa) (ES); Duffaut, Norbert Charles, F-33720 Podesac, Bordeaux (FR); Dunogues, Jacques Paul, F-33400 Talence, Bordeaux (FR); Picard, Jean Paul, F-33400 Talence, Bordeaux (FR); Ganboa Landa, José Ignacio, E-20730 Azpeitia (Gipuzkoa) (ES); Aizpurua Iparraguirre, Jesus Maria, Usurbil, (Gipuzkoa) (ES)
(74) Representative: Curell Sunol, Jorge

(56) References cited:
- FR-A- 2 418 805
- FR-A- 2 536 995
- US-A- 4 322 402
- CHEMICAL ABSTRACTS, vol. 105, no. 21, 24th November 1986, pages 26, 27, abstract no. 183495u, Columbus, Ohio, US; F. SUZUKI et al.: "Antiviral effect of carboxyethylgermanium sesquiocide (GE-132) in mice infected with a lethal dose of influenza virus"
- MEDICAL HYPOTHESES, vol. 26, no. 3, June 1988, pages 207-215, Longman Group, GB; S. GOODMAN: "Therapeutic effects of organic Germanium"
- DRUGS OF FUTURE, vol. 13, no. 5, 1988, pages 441-453; K. MIYAO et al.: "Carboxyethylgermanium sesquioxide and related organogermanium compounds - unique synthetic BRM's - A review"
- PATENT ABSTRACTS OF JAPAN, vol. 5, no. 45 (C-48)[717], 25th March 1981; & JP-A-55 167 222

## Description

The present invention relates to compositions for therapeutic use, useful for the treatment of viral diseases (particularly herpes, shingles, influenza and viral hepatites), and diseases derived from immunitary system deficiencies, such as allergy phenomena in general, asthma and rheumatoid arthritis.

The use of various organogermanium compounds in human and animal therapy has been known for several years, as taught particularly in US Patent 3,812,113 (1974); Rice, L.M., Wheeler, I.W., Gescheckter, C.F., J. Heterocycl. Chem. 11, 1041 (1974) and Japanese patents 46/2946 (1976), 54/115324 (1979). Moreover, water-soluble gemanium containing-polymers having 1,2-substituted 2-carbonyl-ethyl-germanol skeleton of general formula: 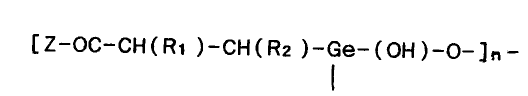
have been described in: French Patent FR 2,418,805(1979), US patent US 4,322,403 (1982), and Miyao K. et al. DRUGS OF FUTURE, 13, no. 5, 1988, pages 441-453.

The last document discloses the antitumor, antiviral and immunostimulatory effect of carboxyethylgermanium sesquioxide (Ge-132) and other structurally related derivatives. This document further indicates that Ge-132 is soluble in water and that in solution Ge-132 is hydrolysed and dissociated to (OH)₃GeCH₂CH₂COOH.

The composition of the invention is characterised in that it comprises in an aqueous solution at least one organogermanium derivative of formula: 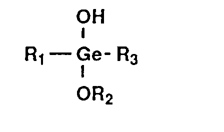
where:
- R₁ is CH₃-, CH₃CH₂- or CH₃CH₂CH₂- or a cyclic aliphatic or heteroaliphatic group, or an aromatic or heterocyclic or heteroaromatic group;
- R₂ is H, R₁ or an atom of lithium, sodium or potassium;
- R₃ is OH, OR₂ or: 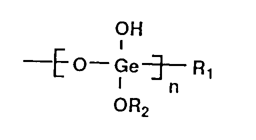
where n is an integer from 0 to 1000, preferably from 0 to 50, the organogermanium compound being used at a concentration ranging from 10⁻¹⁰ and 10⁻² atoms-gram of germanium per litre of water and, more particularly, from 10⁻⁸ to 10⁻³ atoms-gram per litre of water.

The said derivative may be prepared by hydrolysis of chlorinated, brominated, iodated, aminated or alcoxylated organogermanium compounds. When halogenated organogermanium compounds are hydrolysed, the solutions may be purified by heating at atmospheric pressure, to remove the released acids or, as required, at reduced pressure after flowing through Dowex-1 type ionic resins.

When aminated organogermanium compounds are hydrolysed, they may be purified by flowing through Dowex-50 W type cationic resins.

According to the invention, the composition also comprises small amounts of formic acid or one of the sodium, potassium, lithium, magnesium, calcium or trialkylammonium salts thereof, the concentration used ranges from 10⁻¹⁰ to 10⁻⁴ moles per litre of water and, preferably, from 10⁻⁷ to 10⁻⁵ moles per litre of water. Prior to use, the formic acid or salts thereof are purified.

The composition may also comprise small amounts of sodium, potassium, lithium, calcium or magnesium thiosulphate and the concentration used ranges from 10⁻¹⁰ to 10⁻² (preferably from 10⁻⁵ and 10⁻³) moles per litre of water.

In one development of the invention, the solution has a pH ranging from 3 to 8 and more particularly from 5 to 7. To adjust the pH, a sodium or potassium bicarbonate solution may be used.

These solutions may be used intramuscularly or endovenously, but local application on the affected area (where the complaint is localised) gives clinically excellent results.

Nevertheless, the cotton wool compress impregnated with the organogermanium solution may be applied at any place on the body.

From the practical point of view, a cotton wool compress (approximately 5 x 5 cm) impregnated with the mixture may be used. It is placed on the painful or affected area and covered thereafter with a sheet of plastics material (about 15 x 15 cm) to retain the moisture.

The duration of the application is from 8 to 20 hours per day. The daily applications generally last from 7 to 30 days.

The same solutions may be applied by ionokinesis with a conventional d.c. generator (current ranging from 5 to 15 milliamperes).

The electrode connected to the generator negative pole is wrapped in cotton wool impregnated in the organogermanium solution, is applied to the affected area and is held in contact with the skin by a bandage.

The other electrode, connected to the generator positive pole, may be applied to any part of the body or simply held in the hand, while ensuring that the contact area is as large as possible.

It should be noted that a treatment may require from 1 to 30 sessions, but more frequently from 1 to 10, spaced apart in the latter case by 24 to 72 hours.

### Tests on the stability of organogermanium compositions

An organogermanium compound solution was prepared by hydrolysing 0.1 g of CH₃GeI₃ in 10 ml of deuterated water at 20°C, with the deuterium iodide being removed by exchange with Dowex-1 anionic resins. Thereafter, respective groups of two 1 ml fractions of said solution were subjected to acidulation by addition of a 30% deuterium chloride solution in deuterated water and buffering inorganic salts to the following pH values: 6.8, 5.4, 4.1 and 3.2. The two samples of each group were held at temperatures of 20°C and 45°C for two weeks, each sample being anaylsed daily by nuclear magnetic resonance (¹H-NMR) at 300 MHz. The results obtained show that no degradation was observed at the end of the said period.

### Biological activity tests

### Test 1: Production of antibodies (ANTI-GHF-1) in preimmunised rabbits

0.47 g of CH₃GeI₃ were hydrolysed in a 100 ml graduated test tube and the hydrogen iodide formed was removed by treatment with Dowex-1 ionic resins until neutral pH.

Two rabbits of 1.2 kg body weight, preimmunised with GHF-1 protein (specific transcription factor of anterior pituitary, present in GC culture cells) were injected subcutaneously with the antigen. One was administered the above described organogermanium compound solution (topical skin application of 2 ml of solution) according to the invention and the other was used as control.

The product was administered every three days for 10 and 20 sessions. The GHF-1 antigen was reinjected after 20 days.

After 30 days (10 sessions) and 60 days (20 sessions), serum (15 ml) was drawn from both rabbits and the specific antiserum (anti-GHF-1) titration was determined by three different methods: a) "Dot-Blot" (on nitrocellulose paper, using purified GHF-1 as control), b) immunofluorescence on fixed, permeabilised GC cells, and c) immunoprecipitation in ³⁵S-methionine tagged GC cells.

The titration was defined as the maximum dilution producing a positive reaction.

Under these conditions, the control rabbit has the following titrations at 30 days: 200 (method a), 400 (method b), 250 (method c), while the rabbit treated with the organogermanium compound of the invention exhibited the following values: 1000 (method a), 1200 (method b) and 1000 (method c).

Likewise, the titrations of the control rabbit after 60 days were: 400 (method a), 600 (method b) and 400 (method c), while the values of the rabbit treated with the organogermanium compound were: 3000 (method a), 2400 (method b) and 2000 (method c), thereby confirming the overactivation of the immune response (synthesis of immunoglobulins) in the presence of the organogermanium compound.

### Test 2: Production of macrophages in mice

15 BALB/C mice (18-20 g) were divided into two groups of 5 and 10 mice. The first group was administered 1 ml of the aqueous solutions described below every 3 days for 30 days (10 sessions), subcutaneously.

The same was done with the second group, with duplicated results, for 60 days (20 sessions).

The solutions applied were as follows: a) distilled water, b) sodium thiosulphate (25 g/lit), c) formic acid (0.3 g/lit), d) above described organogermanium compound solution, and e) above described organogermanium compound plus formic acid (0.3 g/lit).

At the stated times, the macrophages obtained from the mice peritoneum were purified, cultured and the concentration per millilitre thereof was determined. An amount of 1 x 10⁶ macrophages/ml is deemed to be standard in normal mice, free from latent infection.

The counts obtained after 30 days for the first group treated with solutions a)-e) (in millions of microphages per millilitre) were, respectively: a) 1.0, b) 0.9, c) 1.2, d) 3.2 and e) 5.3.

Likewise, the average counts at 60 days for the second group mice with solutions a)-e) (in millions of macrophages per millilitre) were, respectively: a) 1.1, b) 1.0, c) 1.2, d) 4.2, e) 6.3.

Finally, the macrophages removed from the second Group mice were cultured "in vitro" for 24 hours, protein synthesis (using ³⁵S-met. as indicator) and DNA synthesis (using ³H-Thy.) determinations being made therewith.

If the macrophages from the mice treated with water (subgroup a) are taken as reference (100%), the relative (percentage) values of protein synthesis are: b) 98%, c) 115%, d) 121%, e) 110%, showing that all are metabolically active.

Likewise, the relative (percentage) values of DNA synthesis, showing the activity in cell division, are as follows: a) 100% (reference), b) 102%, c) 140%, d) 125%, e) 150%.

### Test 3: Determination of the interleukin-1 (IL-1) concentration in cell cultures in the presence of organogermanium compound

A line of mouse macrophages P338D1 (reference TIB 63, ATCC catalogue) secreting IL-1 in the culture medium was used.

The cells were cultured for 48 or 72 hours under standard sterility, temperature and CO₂ conditions, in the presence of the organogermanium compound (1 g/lit), prepared as described in Test nº1 plus formic acid (0.3 g/lit).

The IL-1 was determined by the "Western Blot" technique, with an IL-1 specific polyclonal antibody (IP-110, GENZYME CORP. Boston).

Taking the IL-1 levels present in cells treated only with a formic acid solution (0.3 g/lit) as reference (100%), the results for the cells treated with the organogermanium compound plus formic acid were: a) Experiment 1 (48h), IL-1 level: 200%; b) Experiment 2 (72h), IL-1 level: 250%.

### Acute toxicity preclinical tests

All the tests described hereinafter were carried out with doses having concentration levels 10⁴ to 10⁵ times the therapeutical doses.

### - Intravenous administration tests

The administration of doses of 5, 10, 15 and 20 ml of a 3 x 10⁻³ M organogermanium compound solution to four groups of 3 laboratory rabbits (1.2 kg body weight) did not cause dysfunctions or any acute toxicity symptom. The liver and lung post mortems were normal.

### - Oral administration tests

Doses of 10, 15, 20 and 25 ml/day of an identical solution to the above were administered.

The solution was administered with a syringe for 3 days to four groups of 3 laboratory rabbits.

No mortality was observed in any case, although there were symptoms of passing dysfunctions (loss of balance) in the 20 and 25 ml/day groups. All the post mortems were normal.

### Examples of clinical application

EXAMPLE 1: A female patient suffered from repeated herpes attacks on the lower lip for many years with a frequency of two attacks monthly.

The affected area received local applications every hour, with the aid of cotton wool soaked in a solution of the compound according to the invention, prepared as follows:
700 ml of the aqueous solution of the organogermanium compound obtained by hydrolysis of 0.34 g of CH₃GeI₃ were placed in a one litre graduated flask. The majority of the hydriodic acid released by hydrolysis was removed by boiling at a low pressure of 20 mmHg.

The aqueous solution was purified by passing through Dowex-1 anionic resins, followed by washing of the resins with distilled water. Thereafter 0.015 g of formic acid was added. The pH was adjusted to 4.8 by addition of sodium bicarbonate and the solution was topped up to 1 litre with distilled water.

Finally, 5 g of sodium thiosulphate were added to the mixture.

A reduction of the inflammatory oedema was observed in the first hour of application to the patient, followed by quick drying of the skin.

Two applications were made daily by the patient for two days. Subsequently two local applications were made every two months.

No relapse was observed after one year.

EXAMPLE 2: A 45 year old male patient had a sharp temperature rise to 39.9°C, accompanied by all the symptoms of influenza (shivering, muscular pains, palpitations, headache, asthenia).

The inner forearms were rubbed with cotton wool soaked in the composition of the invention, prepared as follows:
700 ml of the aqueous solution of the organogermanium compound obtained by hydrolysis of 0.34 g of CH₃GeI₃ were placed in a one litre graduated flask.

The majority of the hydriodic acid released by hydrolysis was removed by boiling at a low pressure of 20 mmHg.

The aqueous solution was purified by passing through Dowex-1 anionic resins, followed by washing of the resins with distilled water.

Afterwards, a cotton wool compress soaked in the same solution was applied to the patient's chest.

The compress was covered with a sheet of plastics material of 15 x 15 cm, to retain the moisture.

The compress was left in place on the patient's chest for 12 hours.

At the end of this time, all the influenza symptoms had disappeared. The patient's temperature was down to 37.3°C.

Treatment was continued for two days and the temperature was down to normal in a stable fashion.

EXAMPLE 3: A 62 year old male patient had been suffering from a vesicular eruption on the right semithorax for several days, accompanied by great burning pain.

A cotton compress, soaked in a solution of the composition according to the invention and prepared as below was placed on the spinal column, between the shoulder blades.

700 ml of the aqueous solution of the organogermanium compound obtained by hydrolysis of 0.34 g of CH₃GeI₃ were placed in a one litre graduated flask.

The majority of the hydriodic acid released by hydrolysis was removed by boiling at a low pressure of 20 mmHg.

The aqueous solution containing the organogermanium compound was purified by passing through Dowex-1 resins, followed by washing of the resins with distilled water.

The pH was adjusted to 4.8 by addition of sodium bicarbonate and the solution was topped up to 1 litre with distilled water.

Thereafter, 5 g of sodium thiosulphate were added to the solution.

The cotton compress applied between the shoulder blades was covered with a 15 x 15 plastics material sheet to retain the moisture.

This compress was left in place for 12 hours daily.

The pain disappeared during the time following the application of the compress.

The eruption dried rapidly.

No pain sequel remained.

Treatment was continued for five days.

EXAMPLE 4: A 29 year old female patient who has been suffering from asthma from the age of 9, suffered from very violent night crises, requiring the repeated use of beta-stimulants and a corticoid based treatment.

The patient used the composition of the invention as per Example 3.

By associating twice-daily nasal instillations of this composition with the application of a cotton wool compress soaked in the same composition to the chest, covered with a 15 x 15 cm plastics material sheet for 10 hours a day, a fast recession of the crises was observed, allowing the patient to enjoy normal sleep again.

The nasal instillations and the local chest applications continued for a fortnight.

No relapse was observed after one year.

Nevertheless, the patient has had 5-day maintenance treatments every 4 months.

EXAMPLE 5: A 46 year old male patient had a sudden temperature rise accompanied by obvious icterus. The patient was very depressed, with black urine and a lot of foam. Biological examinations gave the following results:
Transaminases S.G.O.T.: 1200; S.G.P.T.: 1140.
Gamma GT.: 400; H.B.S. antigen is positive.

The inner forearm was rubbed with cotton wool soaked in the composition of the invention, prepared as described in Example 3.

Afterwards, a cotton wool compress soaked in the same solution was applied over the liver. The compress was covered with a 15 x 15 plastics material sheet to retain the moisture.

The compress was left in place for 12 hours daily.

After 6 days, the analysis results were:
Transaminases S.G.O.T.: 80; S.G.P.T.: 50.
Gamma GT.: 60.

Clinically, the icterus remitted quickly, the urine became progressively more transparent and the patient's general state improved.

After six months, the patient's general state was very good.

No sequels or relapses have been observed.

EXAMPLE 6: A 51 year old patient complained of a skin allergy with intense itching on the flanks, which kept him awake at night.

Allergological examinations did not allow the allergens to be defined and only an antihistaminic treatment provided momentary relief.

The patient used the composition of the invention as per Example 1.

The twice daily nasal instillations of the composition were accompanied by rubbing the inner forearms twice daily and the application on the chest of a cotton wool compress soaked in the same composition and covered with a 15 x 15 cm plastics material sheet for 8 hours daily.

The skin allergy attenuated rapidly over seven days, without reappearing in one year.

The patient has followed a maintenance treatment of two days every four months.

EXAMPLE 7: A 60 year old woman had been suffering from traditional rheumatoid arthritis for 20 years, with episcleritis and Sjogren's syndrome.

During this time she had been treated with penicillamine, gold salts and finally with various specifics, without the disease having been stopped.

Blood tests before and after these treatments gave the following sedimentation rate: 86-124.

The treatment consisted of applying the composition of the invention as per Example 1 to the elbows and knees for 12 hours daily.

Three weeks after starting treatment, blood tests gave the following sedimentation rate: 45-75. Furthermore, an evident improvement in the motility of the upper and lower limbs was observed.

## Claims

1. A composition for therapeutic use, useful for the treatment of viral diseases, particularly herpes, shingles, influenza and viral hepatites, and diseases derived from immunitary system deficiencies, such as allergy phenomena in general, asthma and rheumatoid arthritis, characterised in that it comprises in an aqueous solution at least one organogermanium derivative of formula: 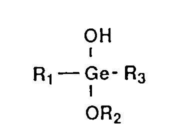 where:
- R₁ is CH₃-, CH₃CH₂- or CH₃CH₂CH₂- or a cyclic aliphatic or heteroaliphatic group, or an aromatic or heterocyclic or heteroaromatic group;
- R₂ is H, R₁ or an atom of lithium, sodium or potassium;
- R₃ is OH, OR₂ or: 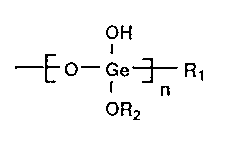
where n is an integer from 0 to 1000, preferably from 0 to 50, the organogermanium compound being used at a concentration ranging from 10⁻¹⁰ and 10⁻² atoms-gram of germanium per litre of water and, more particularly, from 10⁻⁸ to 10⁻³ atoms-gram per litre of water.

2. The composition of claim 1, characterized in that it also comprises small amounts of formic acid or of a salt thereof.

3. The composition of claim 2, characterized in that said salts are the sodium, potassium, lithium, magnesium, calcium or trialkylammonium salt.

4. The composition of claim 2 or claim 3, characterised in that the formic acid or the salts thereof are used at a concentration ranging from 10⁻¹⁰ and 10⁻⁴ moles per litre of water.

5. The composition of any one of the foregoing claims, characterised in that it comprises small amounts of sodium, potassium, lithium, calcium or magnesium thiosulphate.

6. The composition of claim 5, characterised in that said thiosulphates are used at a concentration of ranging from 10⁻¹⁰ to 10⁻² moles per litre of water.

7. The composition of any one of the foregoing claims, characterised in that it has a pH ranging from 3 to 8, more particularly, from 5 to 7.

## Patentansprüche

1. Mittel zur therapeutischen Anwendung, das zur Behandlung von viralen Erkrankungen, insbesondere Herpes, Gürtelrose, Erkältungskrankheiten und viraler Hepatitis sowie von Krankheiten einsetzbar ist, die von Immunsystemdefekten abgeleitet sind, wie Allergiephänomene im allgemeinen, Asthma und rheumatoide Arthritis, **dadurch gekennzeichnet**, daß es in einer wässrigen Lösung mindestens ein Organogermaniumderivat der Formel 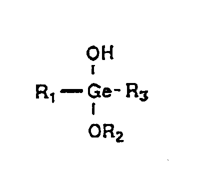 aufweist, in der
R₁ die Gruppe CH₃-, CH₃CH₂- oder CH₃CH₂CH₂- oder eine cyclische aliphatische oder heteroaliphatische Gruppe oder eine aromatische oder heterocyclische oder heteroaromatische Gruppe bedeutet,
R₂ H, R₁ oder ein Lithium-, Natrium- oder Kaliumatom bedeutet,
R₃ OH, OR₂ oder 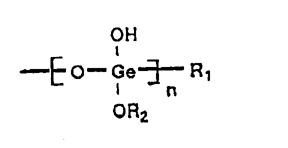 bedeutet, in der n eine ganze Zahl von 0 bis 1000, vorzugsweise von 0 bis 50 ist, wobei die Organogermaniumverbindung in einer Konzentration von 10⁻¹⁰ bis 10⁻² Atomgramm Germanium/l Wasser und insbesondere von 10⁻⁸ bis 10⁻³ Atomgramm/l Wasser eingesetzt wird.

2. Mittel nach Anspruch 1, **dadurch gekennzeichnet**, daß es geringe Mengen an Ameisensäure oder von einem ihrer Salze aufweist.

3. Mittel nach Anspruch 2, **dadurch gekennzeichnet**, daß das Salz das Natrium-, Kalium-, Lithium-, Magnesium-, Calcium- oder Trialcylammoniumsalz ist.

4. Mittel nach Anspruch 2 oder 3, **dadurch gekennzeichnet**, daß die Ameisensäure oder ihre Salze in einer Konzentration von 10⁻¹⁰ bis 10⁻⁴ mol/l Wasser eingesetzt werden.

5. Mittel nach einem der vorhergehenden Ansprüche, **dadurch gekennzeichnet**, daß es geringe Mengen an Natrium-, Kalium-, Lithium-, Calcium- oder Magnesiumthiosulfat aufweist.

6. Mittel nach Anspruch 5, **dadurch gekennzeichnet**, daß die Thiosulfate in einer Konzentration von 10⁻¹⁰ bis 10⁻² mol/l Wasser eingesetzt werden.

7. Mittel nach einem der vorhergehenden Ansprüche, **dadurch gekennzeichnet**, daß es einen pH-Wert von 3 bis 8, vorzugsweise 5 bis 7 aufweist.

## Revendications

1. Composition pour l'utilisation thérapeutique, utile dans le traitement de maladies virales, en particulier de l'herpès, du zona, de la grippe et des hépatites virales, et des affections imputables aux déficiences du système immunitaire telles que des phénomènes d'allergie en général, l'asthme et l'arthrite rhumatismale, caractérisée en ce qu'elle comprend dans une solution aqueuse au moins un dérivé d'organogermanium de formule : 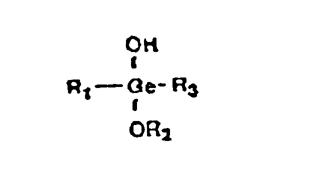 dans laquelle :
- R₁ est CH₃, CH₃CH₂- ou CH₃CH_{z}CH₂- ou un groupe hétéro-aliphatique ou aliphatique cyclique ou un groupe hétéroaromatique ou aromatique ou hétérocyclique ;
- R₂ est H₃ R₁ ou un atome de lithium, de sodium ou de potassium ;
- R₃ est OH, OR₂ ou : 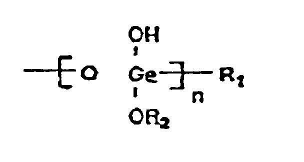
dans laquelle n est un nombre entier de 0 à 1000, de préférence de 0 à 50, le composé organogermanium étant utilisé à une concentration dans la plage de 10⁻¹⁰ et 10⁻² atomes-grammes de germanium par litre d'eau et plus particulièrement, de 10⁻⁸ à 10⁻³ atomes-grammes par litre d'eau.

2. Composition selon la revendication 1, caractérisée en ce qu'elle comprend également de petites quantités d'acide formique ou de son sel.

3. Composition selon la revendication 2, caractérisée en ce que ces sels sont le sel de sodium, de potassium, de lithium, de magnésium, de calcium ou de trialkylammonium.

4. Composition selon la revendication 2 ou la revendication 3, caractérisée en ce que l'acide formique ou ses sels sont utilisés dans une concentration dans la plage de 10⁻¹⁰ et 10⁻¹ moles par litre d'eau.

5. Composition selon l'une quelconque des revendications précédentes, caractérisée en ce qu'elle comprend de petites quantités de thiosulfate de magnésium, de calcium, de lithium, de potassium ou de sodium.

6. Composition selon la revendication 5 caractérisée en ce que ces thiosulfates sont utilisés dans une concentration dans la plage de 10⁻¹⁰ et 10⁻² moles par litre d'eau.

7. Composition selon l'une quelconque des revendications précédentes, caractérisée en ce qu'elle présente un pH dans la plage de 3 à 8, plus particulièrement de 5 à 7.
